(19) Europäisches Patentamt
European Patent Office
Office européen des brevets

(11) Numéro de publication: **0 002 151**
**B1**

(12) # FASCICULE DE BREVET EUROPEEN

(45) Date de publication du fascicule du brevet: **25.05.83**

(51) Int. Cl.³: **C 07 C 69/67**, C 07 C 67/30

(21) Numéro de dépôt: **78400171.1**

(22) Date de dépôt: **10.11.78**

(54) **Procédé de synthèse d'esters parachlorobenzoylphenoxy isobutyriques.**

(30) Priorité: **14.11.77 FR 7734075**

(43) Date de publication de la demande:
**30.05.79 Bulletin 79/11**

(45) Mention de la délivrance du brevet:
**25.05.83 Bulletin 83/21**

(84) Etats contractants désignés:
**BE CH DE FR GB LU NL**

(56) Documents cités:
**FR - A - 2 342 723**

**HOUBEN WEYL "Methoden der organischen Chemie", 1973, E. MUELLER, Tübingen, Band VII-2a-Ketone-Partie 1, pages 311—323**

(73) Titulaire: **DEVINTER S.A.**
**2 Boulevard Royal**
**Luxembourg (LU)**

(72) Inventeur: **Gignier, Jean Pierre**
**4, rue des Capucines**
**F-92190 Meudon (FR)**
Inventeur: **Bourrelly, Jacques**
**4, avenue du Château**
**F-92190 Meudon (FR)**

(74) Mandataire: **Azais, Henri et al,**
**CABINET BEAU DE LOMENIE 55, rue d'Amsterdam**
**F-75008 Paris (FR)**

**EP 0 002 151 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de synthése d'esters parachlorobenzoyl phenoxy isobutyriques

La présente invention concerne la fabrication d'esters 4(parachlorobenzoyl) phénoxyisobutyriques de formule générale:

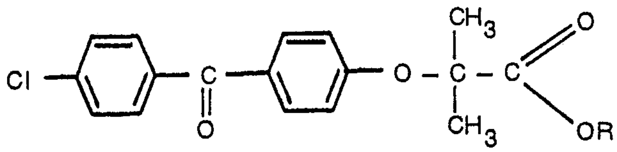

(I)

dans laquelle R est un groupement alkyl inférieur.

Les procédés de fabrication de tels composés décrits à ce jour ont fait l'objet des publications suivantes:

| | |
|---|---|
| BSM | 4433 |
| Brevet français | 1 340 811 |
| Brevet français | 2 035 821 |
| Certificat d'addition | 2 157 853 au brevet français 2 035 821 |
| Brevet français | 2 342 723 |

Les procédés décrits représentent des variantes de la réaction décrite par Bargellini en 1906: action du chloroforme et de l'acétone sur un phénol en milieu alcalindonnant des composés du type "acide clofibrique".

Des composés de formule (I), décrits dans le brevet français 2 035 821 et son addition 2 157 853 sont obtenus par réaction de Bargellini, le dérivé phénolique étant dans ce cas la 4-chloro-4'-hydroxy-benzophénone, correspondant à la formule (II):

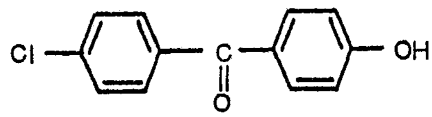

(II)

Le groupement hydroxyle en para d'une fonction carboxylique est peu actif en regard de la réaction chloroforme/acétone et les rendements de condensation sont faibles (75% en acide brut).

La purification de l'acide 4 (parachlorobenzoyl)phenoxy isobutyrique jusqu'à élimination totale du reste de benzophénone est un processus laborieux, mais nécessaire dans le cas de cette synthése. L'estérification de l'acide ainsi obtenu est opérée par des voies classiques.

La synthèse du composé (II) est elle-même laborieuse passant par un réarrangement de Fries à haute température et des purifications difficiles.

Il a paru opportun d'axer les efforts vers l'obtention d'une condensation directe d'un agent d'alcoylation approprié sur un dérivé convenable de l'acide phénoxyisobutyrique (III):

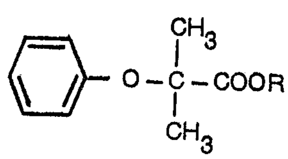

(III)

Ce dérivé (III) est obtenu aisément:

— par réaction de Bargellini phénol plus acétone plus chloroforme et estérification ultérieure.

— par condensation sur le phénate de sodium d'un ester de l'acide alphabromoisobutyrique.

Ces deux voies d'accés sont égalementaisées à partir de réactifs bon marché du commerce.

L'agent d'acylation sélectionné pouvait être de deux types (en présence d'un acide de Lewis comme catalyseur).

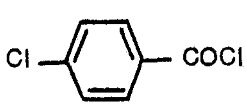

(IV)

Chlorure de l'acide parachlorobenzoique (réaction de Friedel-Crafts)

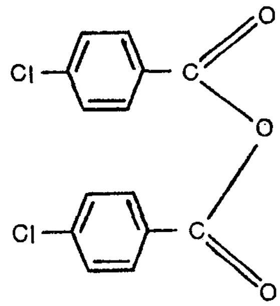

(V)

anhydride de l'acide parachlobenzoïque (condensation Perkin)

Il est apparu lors des essais préliminaires que la condensation Friedel-Craft ne donnait pas des résultats satisfaisants, l'énergie d'activation en position para des esters phénoxyisobutyriques étant insuffisante pour assurer une condensation avec des rendements acceptables quelque soit le catalyseur sélectionné.

Par contre, la réaction de l'anhydride parachlorobenzoique s'est avérée donner des résultats beaucoup plus satisfaisants en présence de divers catalyseurs du type acide de Lewis, notamment $AlCl_3$ et $TiCl_4$, mais les rendements obtenus restent voisins de ceux obtenus par d'autres méthodes.

On a pu par contre, montrer que avec $BF_3$ qui peut être aisément introduit dans le milieu réactionnel et en être ensuite éliminé grâce à sa forme gazeuse, on peut obtenir des rendements essentiellement quantitatifs. Avec ce réactif, on a pu définir des conditions de réactions privilégiées permettant d'atteindre ce résultat. De plus, ces conditions sont douces et la réaction est rapide, ce qui permet une opération économique.

La présente invention est donc relative à un procédé de fabrication d'ester méthylique de l'acide 4 (parachlorobenzoyl) phénoxyisobutyrique, caractérisé en ce que l'on réalise une réaction dite de PERKIN entre l'anhydride parachlorobenzoïque et l'ester méthylique de l'acide phénoxyisobutyrique, ladite réaction étant réalisée au sein d'un solvant en utilisant $BF_3$ comme catalyseur et en opérant à une température voisine de la température ambiante.

Partant de ce procédé, il est possible d'obtenir:

— L'ester méthylique de l'acide 4(parachlorobenzoyl)phénoxyisobutyrique (schéma I)
— L'acide lui-même (schéma 2)
— Un autre ester supérieur par transestérification de l'ester méthylique (schéma 3)
— Un autre ester supérieur par estérification de l'acide (schéma 4)

Schéma 1

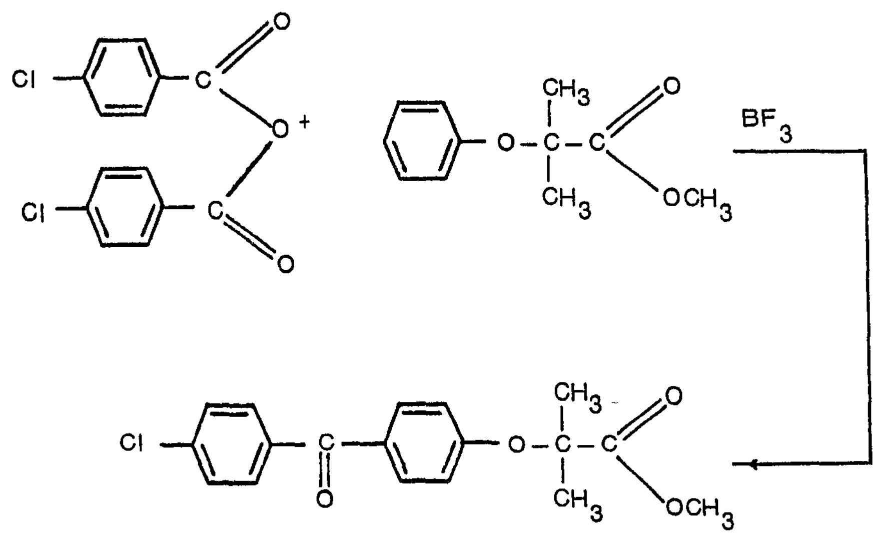

Schéma 2

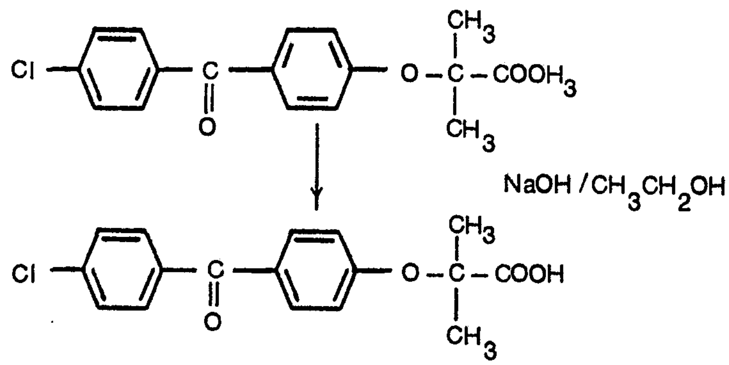


Schéma 3

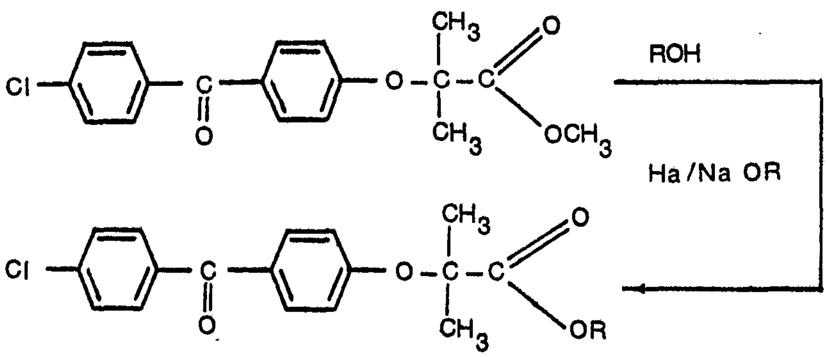


Schéma 4

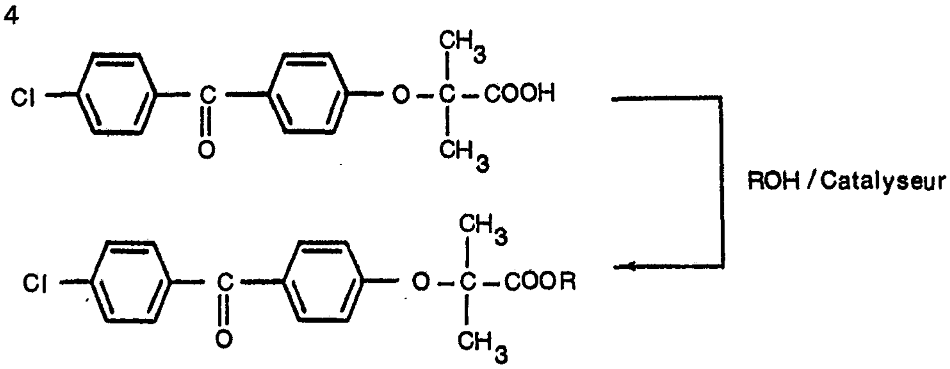


Les exemples ci-après donnent une illustration de l'invention, sur une base non limitative:

Exemple 1 (comparatif)

A 100 ml de chlorure de méthylène on ajoute 17,7 g d'anhydride parachlorobenzoique et 9,7 g de phénoxy isobutyrate de méthyle. On refroidit à 5°C et on ajoute progressivement l mole équivalent de chlorure d'aluminium finement broyé.

On laisse revenir à température ambiante, puis on chauffe au bain marie jusqu'au reflux du chlorure de méthylène. On maintient ainsi pendant 3 heures.

On coule le milieu réactionnel, préalablement refroidi sur de la glace, on alcalinise par de la lessive de soude à 10% et on extrait au chlorure de méthylène. La phase organique est lavée à l'eau puis séchée sur chlorure de calcium.

Après élimination du solvant, on obtient 13,3 g de parachlorobenzoyl phénoxy isobutyrate de méthyl — Rendement 80%. Point de fusion 85°C.

Exemple 2

On mélange sous agitation 125 ml de chlorure de méthylène 22,13 g d'anhydride parachlorobenzoique et 12,1 g de phénoxyisobutyrate de méthyle. On fait ensuite barbotter, à

température ambiante un léger courant de trifluorure de bore, en bullage régulier. On maintient ainsi 3 heures, puis on chauffe jusqu'à ébullition du solvent. On maintient 1 heure au reflux. On refroidit puis on coule le milieu réactionnel sur de la glace. On amène le pH à 10 par addition de lessive de soude à 10%. On extrait au chlorure de méthylène, on lave à l'eau et sèche sur sulfate de magnésium. Après évaporation du chlorure de méthylène, on obtient 15,8 g de parachlorobenzoyl phénoxy isobutyrate de méthyle. Rendement 95%. Point de fusion: 85—86°C.

Exemple 3

On mélange sous agitation 250 ml de chlorure de méthylène, 44,2 g (0,15 mole) d'anhydride parachlorobenzoique et 24,2 g (0,125 mole) de phénoxyisobutyrate de méthyle.

On fait barbotter un leger courant de trifluorure de bore pendant trois heures, à température ambiante, puis on porte au reflux pendant une heure.

Après refroidissement à 0°C et filtration du milieu réactionnel, on récupère 21 g d'acide parachlorobenzoique, soit 90% de la quantité théorique formée.

Le filtrat, évaporé et traité par de la soude alcoolique conduit à 37,7 g (0,118 mole) d'acide 4(4 chlorobenzoyl)phénoxyisobutyrique — Rendement 95% — Point de fusion 187°C.

Exemple 4

On introduit 11,2 g (0,062 mole) d'acide phénoxyisobutyrique, 125 ml de chlorure de méthylène et 22,13 g (0,075 mole) d'anhydride parachlorobenzoique. Après traitement de ce mélange selon le mode opératoire pratiqué à l'exemple 2, on ne décèle par chromatographie aucune trace du produit de condensation attendu.

Exemple 5

27,8 g (0,125 mole) de phénoxyisobutyrate d'isopropyle, 44,2 g (0,15 mole) d'anhydride parachlorobenzoique et 250 ml de chlorure de méthylène, soumis au même traitement que pour l'exemple 2 ont conduit à 8 g de 4 (4 chlorobenzoyl) phénoxyisobutyrate d'isopropyle. Rendement 17,5%.

Exemple 6

12 g d'ester méthylique de l'acide 4 (parachlorobenzoyl) phénoxyisobutyrique sont mélangés avec 0,1 atome équivalent de sodium frais dans 200 ml d'isopropanol. On chauffe à l'ébullition de l'alcool et on maintient ainsi pendant 5 à 6 heures, en éliminant peu à peu le mélange de vapeurs de méthanol et d'isopropanol en tête de reflux. On élimine ensuite le solvant et on reprend par du chlorure de méthylène. En traitant cette phase chlorométhylénique comme pour l'exemple 3, on isole le parachlorobenzoyl phénoxy isobutyrate d'isopropyle brut avec un rendement pratiquement quantitatif. On recristallise ce dernier dans 4 volumes d'isopropanol à 30% d'eau. On obtient ainsi 12,4 g de cristaux blancs. Point de fusion 81°C. Rendement: 95%.

Exemple 7

31,85 g d'acide 4(parachlorobenzoyl)phénoxy isobutyrique 500 ml d'isopropanol et 10 ml d'acide sulfurique sont portés à ébullition pendant 10 heures.

Après concentration sous vide, jusqu'à ce que le volume résiduel atteigne environ 100 ml, le mélange est refroidi à l'ambiance et versé sur 600 g de glace.

Le mélange est alcalinisé avec 40 ml de soude aqueuse à 40% et extrait avec 2 fois 200 ml de chlorure de méthylène.

Après décantation, la couche organique est lavée avec 100 ml d'eau et séchée sur le sulfate de magnésium. Après élimination du chlorure de méthylène, on recueille 31,5 g de 4(parachlorobenzoyl) phénoxyisobutyrique isopropyl ester — Rendement 87%.

**Revendications**

1. Procédé de fabrication d'ester méthylique de l'acide 4(parachlorobenzoyl) phénoxyisobutyrique, caractérisé en ce que l'on réalise une réaction dite de PERKIN entre l'anhydride parachlorobenzoïque et l'ester méthylique de l'acide phénoxyisobutyrique, ladite réaction étant réalisée au sein d'un solvant en utilisant $BF_3$ comme catalyseur et en opérant à une température voisine de la température ambiante.

2. Procédé selon la revendication 1, caractérisé en ce que ledit solvant est un solvant chloré de préférence le chlorure de méthylène.

3. Procédé suivant l'une des revendications 1 et 2, où l'ester méthylique de l'acide 4(parachlorobenzoyl) phénoxyisobutyrique est transformé, par transestérification en autres esters, à savoir des esters d'alkyls supérieurs.

4. Procédé suivant la revendication 3, où l'ester méthylique de l'acide 4-(parachlorobenzoyl) phénoxyisobutyrique est transformé en l'ester isopropylique correspondant.

5. Procédé suivant l'une des revendications 1 et 2 où l'ester méthylique de l'acide 4(parachlorobenzoyl) phénoxyisobutyrique est transformé dans l'acide correspondant par

saponification, ledit acide pouvant être éventuellement estérifié pour donner un ester d'alkyl supérieur.

6. Procédé suivant la revendications 5, où l'ester obtenu à partir de l'acide est l'ester isopropylique.

## Claims

1. A process for manufacturing methyl ester of 4-(parachlorobenzoyl)-phenoxyisobutyric acid, characterized in that a so-called PERKIN reaction is carried out between parachlorobenzoic anhydride and the methyl ester of phenoxyisobutyric acid, said reaction being carried out within a solvent using $BF_3$ as a catalyst and by operating at a temperature close to ambiant temperature.

2. A process according to claim 1, characterized in that said solvent is a chlorinated solvent, preferably methylene chloride.

3. A process according to claim 1 and 2, where the methyl ester of 4(parachlorobenzoyl) phenoxyisobutyric acid is converted into other esters by transesterification, namely into higher alkyl esters.

4. A process according to claim 3, where the methyl ester of 4-(parachlorobenzoyl) phenoxyisobutyric acid is converted into the corresponding isopropyl ester.

5. A process according to any one of claims 1 and 2, where the methyl ester of 4-(parachlorobenzoyl) phenoxyisobutyric acid is converted into the corresponding acid by saponification, said acid being optionally esterified to give a higher alkyl ester.

6. A process according to claim 5, where the ester obtained from the acid is isopropyl ester.

## Patentansprüche

1. Verfahren zur Herstellung von 4-(p-Chlorbenzoyl)-phenoxyisobuttersäure-methylester, dadurch gekennzeichnet, daß eine Perkin-Reaktion zwischen p-Chlorbenzoesäureanhydrid und Phenoxyisobuttersäure-methylester durchgeführt wird, wobei die Reaktion in einem Lösungsmittel unter Verwendung von $BF_3$ als Katalysator durchgeführt und bei einer Temperatur nahe der Umgebungstemperatur gearbeitet wird.

2. Verfahren nach Anspruch 1, dadurch gekennzeichnet, daß das Lösungsmittel ein chloriertes Lösungsmittel, vorzugsweise Methylenchlorid ist.

3. Verfahren nach einem der Ansprüche 1 und 2, wobei der 4-(p-Chlorbenzoyl)-phenoxyisobuttersäure-methylester durch Umesterung in andere Ester, nämlich höhere Alkylester, umgewandelt wird.

4. Verfahren nach Anspruch 3, wobei der 4-(p-Chlorbenzoyl)-phenoxyisobuttersäure-methylester in den entsprechenden Isopropylester umgewandelt wird.

5. Verfahren nach einem der Ansprüche 1 und 2, wobei der 4-(p-Chlorbenzoyl)-phenoxyisobuttersäure-methylester durch Verseifung in die entsprechende Säure umgewandelt wird, welche Säure gegebenenfalls verestert werden kann, um einen höheren Alkylester zu ergeben.

6. Verfahren nach Anspruch 5, wobei der über die Säure erhaltene Ester der Isopropylester ist.